Europäisches Patentamt

European Patent Office

Office européen des brevets

(11) Veröffentlichungsnummer: **0 354 440 B1**

# (12) EUROPÄISCHE PATENTSCHRIFT

(45) Veröffentlichungstag der Patentschrift: **12.07.95**

(21) Anmeldenummer: **89114142.6**

(22) Anmeldetag: **01.08.89**

(51) Int. Cl.⁶: **C12N 7/00**, C12N 15/37,
C07K 14/01, C12P 21/00,
G01N 33/577, G01N 33/569,
C12Q 1/68, C12Q 1/70,
A61K 39/12

(54) Humaner Papillomvirus Typ 57, seine DNA und die davon kodierten Proteine.

(30) Priorität: **06.08.88 DE 3826793**

(43) Veröffentlichungstag der Anmeldung:
**14.02.90 Patentblatt 90/07**

(45) Bekanntmachung des Hinweises auf die
Patenterteilung:
**12.07.95 Patentblatt 95/28**

(84) Benannte Vertragsstaaten:
**AT BE CH DE ES FR GB IT LI LU NL SE**

(56) Entgegenhaltungen:
**EP-A- 0 294 659**

**CHEMICAL ABSTRACTS, Band 111, Nr. 9, 28.
August 1989, Seite 419,Zusammenfassung
Nr. 74472n, Columbus, Ohio, US; E.M. DE
VILLIERS et al.: "Two newly identified human
papillomavirus types (HPV 40 and 57) isolated frommucosal lesions", & VIROLOGY
1989, 171(1), 248-53**

(73) Patentinhaber: **BEHRINGWERKE Aktiengesellschaft**
**Postfach 1140**
**D-35001 Marburg (DE)**

(72) Erfinder: **de Villiers, Ethel-Michele**
**Endweg 14**
**D-6945 Hirschberg (DE)**
Erfinder: **Hirsch-Behnam, Anja**
**Ringstrasse 27**
**D-6900 Heidelberg (DE)**
Erfinder: **zur Hausen, Harald, Prof. Dr. Dr.**
**Unterer Häuselbergweg 8**
**D-6945 Hirschberg (DE)**

Rank Xerox (UK) Business Services
(3.10/3.09/3.3.3)

**Beschreibung**

Die humanen Papillomviren (HPV) bilden eine Gruppe von über 50 verschiedenen Typen. HPV wurde im Zusammenhang mit benignen (Warzen, Kondylome im Genitalbereich) und maligenen (Karzinome der Haut und der Scheide) epithelialen Neoplasmen aufgefunden. Papillomviren lassen sich nicht in Kultur vermehren. Die Verwendung der Humanen Papillomvirus Typ 57 DNA (HPV 57 DNA) als Diagnostikum sowie die Gewinnung der Expressionsprodukte, deren Verwendung als Antigene, die Isolierung von Antikörpern und die Herstellung entsprechender Diagnostika und Therapeutika setzt somit gentechnische Verfahren voraus.

Der Erfindung liegt die erstmalige Isolierung von HPV 57, eine teilweise Charakterisierung seines Genoms sowie Klonierung in pUC 19 zugrunde. Hierdurch wird ein Zugang zur Diagnose von Tumoren (Oral-, Genital- und Hauttumoren) eröffnet, die mit HPV 57 assoziiert sind.

Die Erfindung ist in den Patentansprüchen definiert. Weitere Ausgestaltungen der Erfindung sind im folgenden näher beschrieben.

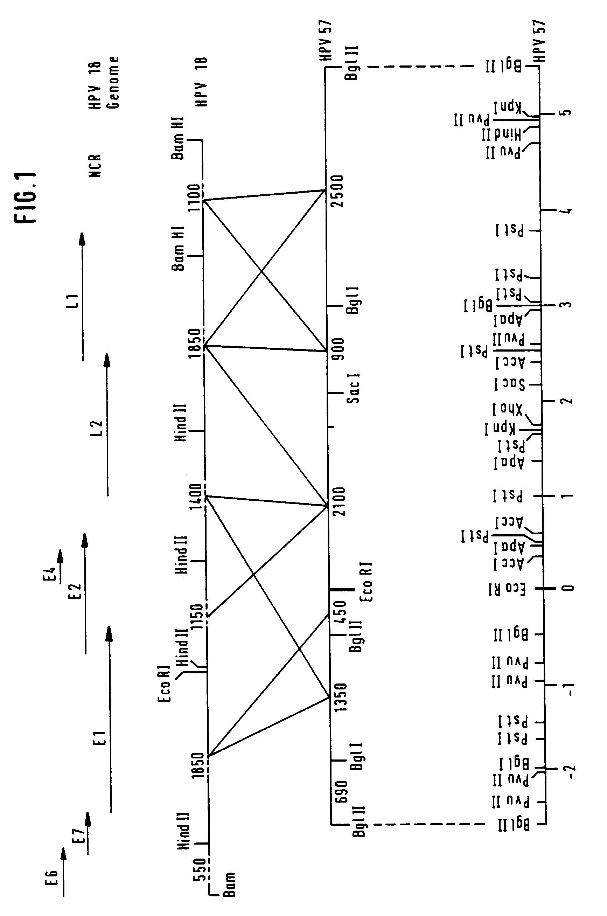

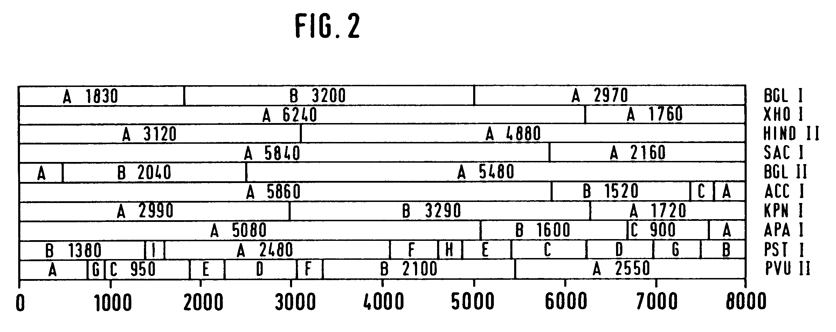

Die Klonierung von HPV 57 ermöglichte den Vergleich mit 56 anderen HPV. Außerdem wurde die erfindungsgemäße HPV 57-DNA mit DNA unterschiedlicher HPV-Typen gemäß EP-A-0 294 659 (Zwischenliteratur) auf der Basis von Restriktionsenzymanalysen verglichen. Dieser Vergleich ergab, daß HPV 57-DNA signifikant verschieden von der DNA der anderen HPV-Typen war. HPV 2 und HPV 27 sind sehr nahe verwandt und durch Hybridisierung in Flüssigphase konnte eine 17%ige bzw. 25%ige Homologie festgestellt werden. Die Kolinearität von HPV 57 mit HPV 2 und 27 wurde bestimmt (Fig. 1) und eine physikalische Genomkarte für Restriktionsenzymspaltungen (Fig. 2) wurde erstellt.

Damit ist ein Zugang geschaffen worden, Neoplasien (Genital- und Hauttumore), insbesondere Tumore im Kopfbereich auf das Vorkommen von HPV 57 zu prüfen und, über Antikörper zu HPV 57 Proteinen, gegebenenfalls therapeutisch anzugehen.

Beispiele

1. Isolierung episomaler HPV 57 DNA

Hochmolekulare DNA wurde aus einem invertierten Papillom der Oberkieferhöhle isoliert wie beschrieben (Gissmann et al. (1982), Int. J. Cancer 29, 143 - 146).

2. Klonierung von HPV 57 in Plasmid pUC 19

Als Klonierungsvektor wurde das bekannte Plasmid pUC 19 (Yanish-Perron et al. (1985), Gene 33, 113 - 119) gewählt. HPV 57-haltige zelluläre Doppelstrang DNA wurde nach Spaltung mit Eco RI in pUC 19 einkloniert. (Maniatis et al.(1982), Molecular Cloning: A Laboratory Manual; Cold Spring Harbor Laboratory Press, New York). Rekombinante Klone wurden im $\beta$-Galactosidase-Test (Messing et al. (1977), Proc. Nat. Acad. Sci. USA 75, 3641 - 3646) identifiziert und einligierte DNA-Stücke nach DNA Schnellextraktion analysiert (Birnboim, H.L. und Doly. (1979), Nucl. Acids. Res. 7, 1513 - 1523).

3. Physikalische Genomkarten von HPV 57

Vom Vektor durch Eco RI-Spaltung abgetrennte HPV 57 DNA wurde mit Restriktionsendonukleasen verdaut und nach allgemein bekannten Methoden die entsprechenden physikalischen Genomkarten erstellt. Das Ergebnis ist in Fig. 2 zusammengefaßt, wobei die alleinige Eco RI-Schnittstelle dazu diente, das HPV 57 Molekül zu linearisieren. Die Länge der einzelnen Restriktionsfragmente zeigt die Tabelle.

4. Vergleich mit anderen HPV

Die DNA des HPV 57-Genoms wurde mit den DNAs von 56 erhältlichen HPV-Typen mittels DNA/DNA Hybridisierung unter verschiedener Stringenz verglichen (E.M. Southern (1975), J. Mol. Biol. 98, 50-517). Unter Bedingungen hoher Stringenz (Schmelztemperatur Tm -20°C) hybridisiert HPV 57 DNA mit HPV 2 und HPV 27.

Die Kolinearität mit HPV 18 aufgrund von Hybridisierungsversuchen zeigt Fig. 1.

In Kenntnis der HPV 18 DNA-Sequenz (Cole et al. (1987) J. Mol. Biol. 193, 599 - 608) sind die offenen Leseraster von HPV 57 ableitbar und damit die HPV 57 Proteine erhältlich über allgemein bekannte Methoden zur Subklonierung mit anschließender Expression in prokaryontischen oder eukaryontischen Expressionssystemen.

Die pUC 19 Plasmid DNA enthaltend HPV 57 ist am 13.6.1988 bei der Deutschen Sammlung für Mikroorganismen unter der Nummer DSM 4694 nach Budapester Vertrag hinterlegt worden.

Legende zu Fig. 2

Das zirkuläre Genom wurde über seine Eco RI-Schnittstelle kloniert, die Zahlenwerte nach den die Fragmente bezeichnenden Buchstaben geben die Länge in Basenpaaren (bp) an. Restriktionsenzyme mit einer Spaltstelle sind XhoI, HindII, SacI. Die Restriktionsenzyme XbaI, SalI, BamHI, HindIII und HpaI spalten nicht. Die exakte Lokalisierung der vier 500 bp langen PstI-Fragmente wurde nicht bestimmt.

Tab.

| | PST I | KPN I | HIND II | BGL II | BGL I | HPA I | ECC I |
|---|---|---|---|---|---|---|---|
| A | 2.400 | 4.710 | 8.000 | 5.960 | 4.800 | 5.500 | 6.220 |
| B | 1.090 | 3.290 | | 2.040 | 3.200 | 1.600 | 1.520 |
| C | .820 | | | | | .900 | .260 |
| D | .710 | | | | | | |
| E | .510 | | | | | | |
| F | .530 | | | | | | |
| G | .520 | | | | | | |
| H | .260 | | | | | | |
| I | .220 | | | | | | |

| | XHO I | SAC I | PVU II |
|---|---|---|---|
| A | 8.000 | 8.000 | 3.290 |
| B | | | 2.100 |
| C | | | .950 |
| D | | | .800 |
| E | | | .390 |
| F | | | .200 |
| G | | | .190 |

**Patentansprüche**

1. Isolierte HPV 57 DNA, gekennzeichnet durch das Restriktionsmuster gemäß Fig. 2, sowie Mutanten und Varianten, die für ein funktionelles HPV 57 kodieren.

2. HPV 57 DNA in pUC 19 mit der Hinterlegungsnummer DSM 4694.

3. Isoliertes HPV 57, einschließlich seiner Mutanten und Varianten, gekennzeichnet durch die HPV 57 DNA gemäß Anspruch 1 oder 2.

4. Verfahren zur gentechnischen Expression von DNA gemäß Anspruch 1 oder 2.

5. Verwendung der DNA gemäß Anspruch 1 oder 2 zum Nachweis von HPV 57 DNA oder RNA in Patientenproben durch Hybridisierung.

6. Diagnostikum enthaltend DNA nach Anspruch 1 oder 2 oder HPV 57-spezifische Sequenzen dieser DNA.

7. Verwendung von HPV 57 gemäß Anspruch 3 zur Herstellung einer Vaccine.

8. Verfahren gemäß Anspruch 4 zur Herstellung einer Vaccine.

**Claims**

1. Isolated HPV 57 DNA, characterized by the restriction pattern shown in Fig. 2, and mutants and variants which code for a functional HPV 57.

2. HPV 57 DNA in pUC 19 with the deposit number DSM 4694.

3. Isolated HPV 57, including its mutants and variants, characterized by the HPV 57 DNA as claimed in claim 1 or 2.

4. A process for genetically engineered expression of DNA as claimed in claim 1 or 2.

5. The use of the DNA as claimed in claim 1 or 2 for the detection of HPV 57 DNA or RNA in patients' samples by hybridization.

6. A diagnostic aid containing DNA as claimed in claim 1 or 2 or HPV 57-specific sequences of this DNA.

7. The use of HPV 57 as claimed in claim 3 for the production of a vaccine.

8. The process as claimed in claim 4 for the production of a vaccine.

**Revendications**

1. ADN de HPV 57 isolé, caractérisé par le profil de restriction selon la figure 2, et ses mutants et variants qui codent pour un HPV 57 fonctionnel.

2. ADN de HPV 57 dans pUC 19 portant le numéro de dépôt DSM 4694.

3. HPV 57 isolé, y compris ses mutants et variants, caractérisé par l'ADN de HPV 57 selon la revendication 1 ou 2.

4. Procédé pour l'expression par génie génétique de l'ADN selon la revendication 1 ou 2.

5. Utilisation de l'ADN selon la revendication 1 ou 2, pour la détection, par hybridation, d'ARN ou d'ADN de HPV 57 dans des échantillons provenant de patients.

6. Agent de diagnostic contenant de l'ADN selon la revendication 1 ou 2, ou des séquences de cet ADN spécifiques de HPV 57.

7. Utilisation de HPV 57 selon la revendication 3, pour la fabrication d'un vaccin.

8. Procédé selon la revendication 4, pour la fabrication d'un vaccin.

FIG.1

# FIG. 2

| | | | | |
|---|---|---|---|---|
| A 1830 | B 3200 | A 2970 | | BGL I |
| A 6240 | | A 1760 | | XHO I |
| A 3120 | A 4880 | | | HIND II |
| A 5840 | A 2160 | | | SAC I |
| A B 2040 | A 5480 | | | BGL II |
| A 5860 | B 1520 | C A | | ACC I |
| A 2990 | B 3290 | A 1720 | | KPN I |
| A 5080 | B 1600 | C 900 A | | APA I |
| B 1380 I A 2480 | F H E C D G B | | | PST I |
| A G C 950 E D F B 2100 | A 2550 | | | PVU II |

0    1000    2000    3000    4000    5000    6000    7000    8000

EP 0 354 440 B1